# EUROPEAN PATENT APPLICATION

(11) **EP 3 309 242 A1**
(43) Date of publication of application: **18.04.2018**
(21) Application number: 15891816.9
(22) Date of filing: 12.05.2015
(51) Int. Cl.: C11B 11/00, C07F 9/10, C12P 9/00

(54) **METHOD FOR PRODUCING ETHER PHOSPHOLIPIDS**

(71) Applicant: Fujino, Takehiko, Fukuoka-shi, Fukuoka 813-0043 (JP); Mawatari, Shiro, Fukuoka-shi, Fukuoka 813-0016 (JP)
(72) Inventor: Fujino, Takehiko, Fukuoka-shi, Fukuoka 813-0043 (JP); Mawatari, Shiro, Fukuoka-shi, Fukuoka 813-0016 (JP)
(74) Representative: Burger, Hannes
(86) International application number: PCT/JP2015/063617
(87) International publication number: WO 2016/181491

(57) **Abstract**

[PROBLEM TO BE SOLVED]

To provide a method for producing ether phospholipids of high purity by an easy process. [SOLUTION] This production method comprises: (A) a step of extracting total lipids from organisms with non-polar organic solvent and branched alcohol mixture; and (B) a step of reacting the total lipids obtained by the step (A) with phospholipase A1 to decompose mixed diacyl-glycerophospholipids. This makes it possible to produce plasmalogen-type glycerophospholipids or ether phospholipids of high purity by an easy process.

## Description

### FIELD OF THE INVENTION

The present invention relates to a method for producing ether phospholipids. More specifically, the present invention relates to a method for producing highly-purified ether phospholipids by an easy process.

### BACKGROUND OF THE INVENTION

Lipid refers to substances that have a long fatty acid chain or a similar hydrocarbon chain in a molecule and that are present in a living body or derived from a living thing.

The lipids may be classified into storage lipids and structural lipids.

Storage lipids comprise C, H and O, and are generally soluble in acetone. Triacylglycerol that is a storage lipid is present in fat tissues of an animal body as energy storage.

On the other hand, structural lipids contain a lipid-group containing P of phosphoric acid and N of base, etc.

Thus, structural lipids comprise a hydrophobic part (fatty acid part) and a hydrophilic part (phosphoric acid and base part) to exhibit amphipathic property.

Generally, while the storage lipids are soluble in acetone, structural lipids are insoluble in acetone.

Such structural lipids are structural components of a biomembrane.

The structural lipids may be roughly classified into following categories:
(1) Glycerophospholipids;
   Examples include phosphatidylcholine (lecithin), phosphatidylethanolamine, etc.
(2) Phosphosphingolipids;
   Examples include sphingomyelins, ceramide ciliatine, etc.
(3) Glycosphingolipids;
   Examples include cerebrosides, sulfatides, gangliosides, etc. and
(4) Glycoglycerolipids;

Examples include diacylglycerols existing in a microorganism or higher plants, coupled by various sugar.

The above (2) phosphosphingolipids and (3) glycosphingolipids are collectively called as sphingolipids.

The glycerophospholipid is a collective term for phospholipids having a glycerol in their skeleton, examples of which include phosphatidylcholine, phosphatidylethanolamine, phosphatidylserine, etc.

Many of those glycerophospholipids are bound to a non-polar part by an ester bond (acyl bond), and some are bound at the *sn-*1 position of glycerol by a vinyl ether bond (alkenyl bond) or an ether bond (alkyl bond).

The former ones bound by a vinyl ether bond are also called as plasmalogens.

Glycerophospholipids having a vinyl ether bond and those having an ether bond are collectively called as ether phospholipids.

Phospholipids are important as structural components of a biomembrane Approximately 18% of phospholipids of a mammal biomembrane are plasmalogens that are ether phospholipids.
In particular, many of them are found in brain nerves, cardiac muscles, skeletal muscles, white blood cells and sperms.
Many of plasmalogens are bound to polyunsaturated fatty acids such as docosahexaenoic acids, arachidonic acids, etc.

Therefore, they play not only a role as storage of second messengers for signals between cells such as prostaglandin, leukotriene, etc., but also significant roles as cell fusion, ion transport, etc.

In addition, since a vinyl ether bond (alkenyl bond) of plasmalogens is particularly susceptible to oxidative stress, they act as an antioxidant at cell membranes.

In mammals, ether phospholipids having an alkyl bond are present although in a small amount. In particular, it is confirmed that phosphatidylcholine and phosphatidylethanolamine having an alkyl bond are present in a rat's brain hippocampus.

Furthermore, it is known that ingested phospholipids having an ether bond (alkyl bond) are transformed into plasmalogens.

These days, it is reported, as shown in WO2011/083827 (Patent Document 1), that plasmalogens-type glycerophospholipids have an effect of brain neurogenesis.

In addition, as shown in WO2012/039472 (Patent Document 2) and Ifuku et al., Journal of Neuroinflammation, 9:197 (2012) (Non-patent Document 1), it is reported that plasmalogens-type glycerophospholipids inhibit an increase of glia cells that is considered to be one of the causes triggering a central nervous system inflammation, thereby effective for improving a central nervous system inflammation, and that they are particularly effective for preventing and treating neurodegenerative disease such as Alzheimer's disease.

Further, it is reported, as shown in for example Patent Documents 3-5, that such plasmalogens-type glycerophospholipids may be obtained from a bird tissue such as chicken skin and chicken breast, in an easy manner on a massive scale.

### PRIOR ART DOCUMENTS

### PATENT DOCUMENTS

- Patent Document 1: WO2011/083827 (Scope of Claims)
- Patent Document 2: WO2012/039472 (Scope of Claims)
- Patent Document 3: JP2008/179588 (Scope of Claims)
- Patent Document 4: WO2008/146942 (Scope of Claims)
- Patent Document 5: WO2010/047404 (Scope of Claims)

### NON-PATENT DOCUMENTS

- Non-patent Document 1: Ifuku et al., Journal of Neuroinflammation, 9:197 (2012)
- Non-patent Document 2: Yamashita et al., J Oleo Sci, 63, 423-430 (2014)

### SUMMARY OF THE INVENTION

### PROBLEM TO BE SOLVED BY THE INVENTION

Conventionally, in order to obtain highly-purified phospholipids from total lipids contained in foods, animal tissues, etc., a purification method used in general required complicated steps of extracting with chloroform/methanol systems, thereafter separating neutral fat and phospholipids by column chromatography.

Patent Documents 3-5 disclose methods for producing plasmalogens-type glycerophospholipids from chicken tissues such as chicken skin, chicken breast, etc. in an easy manner on a massive scale.

However, those production methods had never been satisfactory as an easy production method of ether phospholipids or plasmalogen-type glycerophospholipids.

Non-patent Document 2 discloses a production method, where the processes are complicated and the purity is not sufficient.

Therefore, it had been constantly sought a method for producing plasmalogens-type glycerophospholipids or ether phospholipids of high purity by an easy manner on a massive scale, which is superior to the conventional ones, in light of effects of treating and improving brain diseases such as Alzheimer's disease, Parkinson disease, depression and schizophrenia, metabolic diseases such as diabetes, various infectious diseases, and immune disorders.

Under such circumstances, the present inventors have studied for an objective to provide a method for producing highly-purified plasmalogens-type glycerophospholipids or ether phospholipids by an easy process.

As a consequence, the present inventors found that, by providing a certain process to an organism, highly purified plasmalogen-type glycerophospholipids or ether phospholipids can be obtained by an easy process. The present invention was so completed.

### SOLUTION TO THE PROBLEM

The present invention according to Claim 1 is characterized in,
a method for producing ether phospholipids from an organism comprising:
(A) a step of extracting total lipids with a non-polar organic solvent and branched alcohol mixture; and
(B) a step of reacting the total lipids obtained by the step (A) with phospholipase A1 to decompose mixed diacyl-glycerophospholipids.

The present invention according to Claim 2 is characterized in,
the method for producing ether phospholipids according to Claim 1,
wherein the non-polar organic solvent and branched alcohol mixture is hexane/isopropanol mixture.

The present invention according to Claim 3 is characterized in,
the method for producing ether phospholipids according to Claim 1 or 2 including
a step of purifying the resulting mixture after the step (B) is provided to get purified ether phospholipids.

The present invention according to Claim 4 is characterized in,
the method for producing ether phospholipids according to Claim 3,
wherein the purifying step is made by solution partitioning.

The present invention according to Claim 5 is characterized in,
the method for producing ether phospholipids according to Claim 4,
wherein the solution partitioning is made by extracting lipids with hexane to separate with acetone or water .

### EFFECTS OF THE INVENTION

According to the present invention, a method for producing ether phospholipids comprises (A) a step of extracting from organisms their total lipids with a non-polar organic solvent and branched alcohol mixture, and (B) a step of reacting the total lipids obtained by the step (A) with phospholipase A1 to decompose mixed diacyl-glycerophospholipids. This production method enables to obtain highly-purified ether phospholipids by an easy process.

In particular, this method for producing ether phospholipids enables to obtain far highly-purified ether phospholipids without using column chromatography but merely by extracting lipids with hexane to separate with acetone or water.

### BRIEF DESCRIPTION OF THE DRAWINGS

FIG. 1 shows HPLC charts, where FIG. 1(a) is an HPLC chart depicting total phospholipids derived from a bivalve (clam), and FIG. 1(b) is an HPLC chart depicting purified ether phospholipids derived from a bivalve (clam).
FIG. 2 shows HPLC charts, where FIG. 2(a) is an HPLC chart depicting total phospholipids derived from a bivalve (corbicula), and FIG. 2(b) is an HPLC chart depicting purified ether phospholipids derived from a bivalve (corbicula).
FIG. 3 shows HPLC charts, where FIG. 3(a) is an HPLC chart depicting total phospholipids derived from a chicken breast, and FIG. 3(b) is an HPLC chart depicting purified ether phospholipids derived from a chicken breast.
FIG. 4 shows HPLC charts, where FIG. 4(a) is an HPLC chart depicting composition percentage of polyunsaturated fatty acids per total fatty acids in ether phospholipids derived from a chicken breast, FIG. 4(b) is an HPLC chart depicting composition percentage of polyunsaturated fatty acids per total fatty acids in ether phospholipids derived from a clam, and FIG. 4(c) is an HPLC chart depicting composition percentage of polyunsaturated fatty acids per total fatty acids in ether phospholipids derived from a corbicula.

### DESCRIPTION OF EMBODIMENTS

The followings describe embodiments to exercise the present invention, a method for producing ether phospholipids.

Although the present invention is described mainly by preferred representative examples, the present invention is not limited to such examples.

According to the present invention, the method for producing ether phospholipids comprises the following steps.

This method enables to obtain plasmalogen-type glycerophospholipids or ether phospholipids by an easy process.

(A) a step of extracting from organisms their total lipids with a non-polar organic solvent and branched alcohol mixture; and
(B) a step of reacting the total lipids obtained by the step (A) with phospholipase A1 to decompose mixed diacyl-glycerophospholipids.

Here, ether phospholipid represents glycerophospholipid that has a vinyl ether bond (alkenyl bond) or an ether bond (alkyl bond) at the 1^{st} position of the glycerol backbone *(sn-*1*).*

General formulas of ether phospholipid are described below. A compound represented by the formula (1) is alkenyl phospholipid (plasmalogens), while a compound represented by the formula (2) is alkyl phospholipid.

Formula (1)

CH₂O-CH=CHR¹ (*sn*-1) CH-O-CO-R² (*sn*-2) CH₂OPO₃-X (*sn*-3) (1)

Formula (2)

CH₂O-CH-CHR¹ (*sn*-1) CH-O-COR² (*sn*-2) CH₂OPO₃-X (*sn*-3) (2)

In the above formulas, R¹ represents an aliphatic hydrocarbon group.

R¹ is usually an aliphatic hydrocarbon group having 14 to 18 carbon numbers.

R² represents an aliphatic hydrocarbon group, which is often cases bound to polyunsaturated fatty acid, such as arachidonic acid (ARA), docosahexaenoic acid (DHA), eicosapentaenoic acid (EPA), and the like.

Further, X represents a polar group in the above formulas.

Preferably, X is ethanolamine, choline, serine, inositol, etc.

In particular, ether phospholipids present in a mammal contain mainly ethanolamine plasmalogens in which X in the above formulas is ethanolamine and choline plasmalogens in which X is choline.

Ingested glycerophospholipids having an ether bond (alkyl bond) are absorbed in the original form to be used in each tissue, or are transformed inside a body into alkenyl bond-type phospholipids (plasmalogens).

According to the present invention, an organism used therein may be anything and not be limited in particular, as long as it contains plasmalogen-type glycerophospholipids or ether phospholipids.

Example may include animals and microorganisms.

The exemplary animals may be mammals, birds, fish and shellfish, etc.

It is preferred that the mammals are domestic animals due to the two aspects: supply stability; and safety. Examples include the mammals such as cows and pigs, as well as horses, goats, sheep, dears, camels, lamas, etc. Further, they may include the domestic poultry such as chicken, ducks, turkeys, ostriches, etc.

With regard to the mammals, primary tissues containing ether phospholipids may include skins, brain, intestines, heart, genitals, etc.

Fish and shellfish are preferred because they are capable of being farmed, or cultivated. Examples may be:
Crustacean such as Japanese tiger prawns, tiger shrimps, fleshy prawns, Japanese blue crabs, etc.

Shellfish such as abalones, horned turbans, scallops, oysters, etc.

Sea urchins, ascidian, sea stars, sea cucumbers, actiniae, etc.

In particular, more preferred examples are bivalves such as clams, corbicula, giant clams, scallops, oysters, etc.

With regard to the bivalves, primary tissues containing ether phospholipids may include internal organs, gonad, muscles, etc.

It is preferred that the microorganism is anaerobic bacterium, and it is particularly preferred bacteria in intestines, for example, bacteria of *Acidaminococcaceae* family, etc.

With regard to bacteria, "biological tissue" is bacteria by itself.

In the said step (A), it obtains total lipids of organisms or their tissues.

Preferably, in the case of using tissues as organisms, they are minced or ground in advance before the use.

Examples of the non-polar organic solvent include saturated aliphatic hydrocarbons, preferably straight-chain saturated hydrocarbon, and more preferably hexane.

Examples of the branched alcohol include secondary alcohol and tertiary alcohols, preferably secondary alcohols, and more preferably isopropanol.

The said step (B) is to remove diacyl-type phospholipids by hydrolyzing diacyl-type glycerophospholipids mixed within the total glycerophospholipids obtained by the step (A).

For the above hydrolysis, phospholipase A1 (PLA1) is used.

This PLA1 specifically hydrolyzes an acyl bond at the *sn*-1 of diacyl-type phospholipids.

Therefore, PLA1 does not act on an ether bond at the *sn*-1 of ether phospholipids.

By the PLA1 processing, diacyl-glycerophospholipids are decomposed into free fatty acids and lyso phospholipids.

Free fatty acids and lyso phospholipids may be removed by harnessing their nature of being relatively water-soluble.

The PLA1 is not particularly limited to its derivation, as long as it can attain the above effect.

The exemplary PLA1 may be the one derived from *Aspergillus orizae.*

Such PLA1 may be purchased from Mitsubishi Kagaku Foods Corporation.

The amount of the PLA1 used may be selected as deemed fit, depending on the amount of the total glycerophospholipids.

The amount per 1g of the total glycerophospholipids is preferably 0.15-0.45g, more preferably 0.2-0.3g.

Enzyme reaction by the PLA1 may be performed in a buffer. Such a buffer may be selected as deemed fit depending on PLA1 used. For example, 0.1M citric acid-HCI buffer (pH4.5) may be used.

In that case, the total glycerophospholipids may be added by the buffer to dissolve, and subsequently PLA1 may be added.

Although there is no particular restriction as to the amount of the buffer used as long as enzyme reaction can be progressed, the amount per 1g of the total glycerophospholipids is preferably 1-30mL, more preferably 5-15mL.

The reaction conditions can be selected as deemed fit.

The reaction is run preferably at the temperature of 30-70°C, more preferably at 45-55 °C, and further preferably at 50°C. Stirring is provided during the reaction. The reaction time is preferably 1-5 hours, and more preferably 1-2 hours.

Then the pH used is preferably pH3.5-5.5, and more preferably pH4-5.

According to the present invention, the production method may further include a step of purifying ether phospholipids.

It is preferred to include the purification step, since the purification step enables to obtain ether phospholipids having far superior effects.

More specifically, a purification step is further added to a process liquid obtained by the step (B) where diacyl phospholipids are decomposed.

The purification may be performed by a publicly-known method.

For example, since ether phospholipids are soluble in hexane but are hardly soluble in a water-soluble ketone series solvent, ether phospholipids may be purified by performing a partition combining these solvents and water as deemed fit, further performing a solution partition by water or water-solution (solvent partition method) to remove lyso phospholipids.

In other words, a water-soluble ketone series solvent such as acetone enables to remove neutral fat other than phospholipids, and to separate between ether phospholipids and lyso phospholipids by a waterborne solution partition.

More specifically, to the hydrolysis process liquid, for example, is added hexane/isopropanol mixture (3:2) that is diluted by 5-10 times, and placed into a separating funnel. It is then added by approximately 2/3 amount of water to separate bilayer, and the upper layer (hexane layer) is recovered. By doing so, a lipid decomposed product (free fatty acid, lyso phospholipid), enzyme protein and enzyme buffer may be removed.

Further, neutral fat other than phospholipids may be removed by water-soluble ketone series solvent such as acetone that is diluted by 20-25 times.

According to the present invention, ether phospholipids obtained by the production method contain mainly ethanolamine phospholipids and choline phospholipids.

The structure of such lipids may be analyzed and confirmed using High-Performance Liquid Chromatography (HPLC) on the ether phospholipids.

Ether phospholipids obtained by the production method having such a structure is able to be significantly effective for treating and improving brain diseases such as Alzheimer's disease, Parkinson disease, depression and schizophrenia, metabolic diseases such as diabetes, various infectious diseases, and immune disorders.

Furthermore, according to the present invention, the production method is able to obtain the ether phospholipids of high purity, in particular 80% or higher purity.

In particular, ether phospholipids derived from bivalve tissues are bound at the *sn-2* position to a number of docosahexaenoic acid (DHA), arachidonic acid (ARA) and eicosapentaenoic acid (EPA).
In other words, since they contain one or more of the kinds as residue selected from a group comprising docosahexaenoyl group, arachidonoyl group and eicosapentaenoyl group, they have significantly superior effects.

Since the ether phospholipids are effective for treating and improving brain diseases such as Alzheimer's disease, Parkinson disease, depression and schizophrenia, metabolic diseases such as diabetes, various infectious diseases, and immune disorders, such effects may be achieved by ingesting them into a living body.

The ether phospholipids may be applied to a beverage and food product as a material and to a pharmaceutical composition as an ingredient.
Such a beverage and food product and a pharmaceutical composition may be manufactured in accordance with a publicly-known method.

Furthermore, the ether phospholipids may be applied to various forms of beverage and food products whether publicly known or that would be developed in the future, as deemed fit.

Similarly, they may be applied to a form of functional foods or that of specified health foods.

The exemplary form of beverage and food products includes a sweet (such as frozen dessert, jelly, cake, candy, chewing gum, etc.), bread, dairy product such as milk, yogurt, etc., and other various products.

By using a seasoning agent and/or sweetening agent that may be used in a beverage and food product, they may be applied in a form of beverage as a liquid solution.

Although it is easy and convenient to use the ether phospholipids by mixing them into a product used, it is as a matter of course that a certain amount of the ether phospholipids is required to achieve the above effects.
For example, a beverage and food product contains the ether phospholipids preferably in the amount of approximately 0.01-80 mass%, more preferably in the amount of approximately 0.05-20 mass%.

### EXAMPLES

The followings describe the present invention with reference to examples in details on a method for producing ether phospholipids.

However, the present invention is not limited to these examples.

### EXAMPLE 1

### (Production of ether phospholipids derived from clams)

### (1) Extraction of total clam lipids

To approximately 200g of clams after removing shells, 1,000mL of hexane/isopropanol mixture (3:2) was added, ground by a blender, and placed for 1 hour at a room temperature while mixing.

Subsequently, suction filtration was provided, and its residue was washed by 200mL of hexane/isopropanol mixture (3:2) and the filtrate was combined, and then placed in a separating funnel.

To the separating funnel, 800mL of water or sodium sulfate aqueous solution (1g of sodium sulfate was dissolved into 150mL of water) was added and mixed, then placed in static standing.

Out of two separate layers, the lower layer was removed, and the upper hexane layer was separated.

The hexane layer so obtained was dried by a rotary evaporator, and total bivalve lipids were obtained.

### (2) Analysis of clam-derived total phospholipids by HPLC

HPLC was performed on the total phospholipids so obtained under the following conditions.

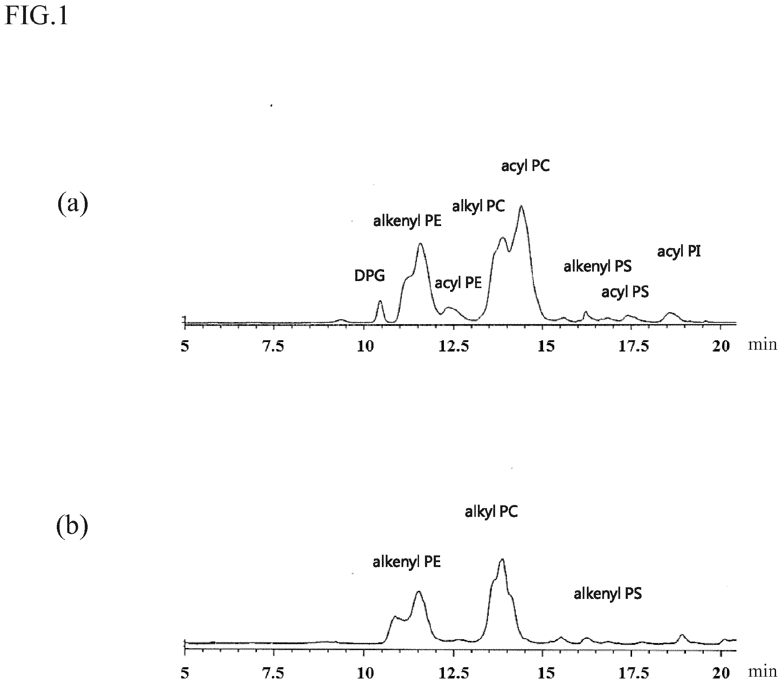

The result is shown in FIG. 1(a).

In this figure, references are as below:
- DPG:: Diphosphoglyceride
- PE:: Ethanolamine phospholipid
- PC:: Choline phospholipid
- PS:: Serine phospholipid
- PI:: Inositol phospholipid
- SM:: Sphingomyelin

### <Conditions for HPLC>

Device used: HPLC Agilent 1100 System (Agilent Technologies, Tokyo)
Column: Lichrosphere 100Diol (250*3mm, 5µm) (Agilent Technologies)
Flow rate: 0.8ml/min
Detection: ELSD (evaporative light scattering detection) (Agilent Technologies)
Mobile phases:
   (A) Hexane/isopropanol/acetic acid (82:17:1, v/v, 0.08%TEA*)
   (B) Isopropanol/water/acetic acid (85:14:1, 0.08%TEA*)
      *TEA: Triethylamine
TABLE 1 shows a gradient of time zone and mobile phases (A) and (B).

**TABLE 1**

| Time (min.) | Mobile phase (A) (%) | Mobile phase (B) (%) |
|---|---|---|
| 0 | 95 | 5 |
| 21 | 40 | 60 |
| 25 | 15 | 85 |
| 26 | 15 | 85 |
| 29 | 95 | 5 |
| 34 | 95 | 5 |

### (3) Purification of ether phospholipids by enzyme treatment and extraction processing of total phospholipids

The total phospholipids so obtained were dispersed in 40mL of phospholipase A1 (Mitsubishi Chemical Foods Corporation) solution (20mg/mL, 0.1M citrate buffer solution (pH4.5)), well mixed using ultra sonic waves, etc., and then was kept for the reaction to run at a temperature of 50°C for 1 hour.

Subsequently, the mixture was cooled and the reaction was stopped.

Then it was added 360mL of hexane/isopropanol (3:2), which was then placed in a separating funnel, mixed, added 200mL of water, and placed in static standing, then a water layer was removed.

A hexane layer was separated, 200mL of water was added, and then mixed.

The upper hexane layer was separated and dried by a rotary evaporator.

Subsequently, in order to remove remaining neutral fat such as cholesterol, the resulting mixture was added by acetone diluted by 20 times, well mixed, and cooled at a temperature of -30°C for 2 hours.

It was then centrifuged 1000 x g for 10 minutes at a temperature of 4°C to separate precipitation. Accordingly, clam-derived purified ether phospholipids (purity: approximately 80%) were obtained.

### (4) HPLC analysis of clam-derived purified ether phospholipids (ether-type glycerophospholipids)

HPLC analysis was provided on the ether-type glycerophospholipids derived from clams (bivalves) so obtained under the above HPLC conditions.

The result is shown in FIG. 1(b).

As a consequence, we succeeded in obtaining highly-purified ether phospholipids derived from clams, where the purity was approximately 80%.

### EXAMPLE 2

### (Production of ether phospholipids derived from corbiculae)

### (1) Extraction of extraction of total corbicula phospholipids

Except that corbicula was used instead of clam, total phospholipids were obtained by the method similar to EXAMPLE 1.

### (2) HPLC analysis of corbicula-derived total phospholipids

HPLC analysis was provided on the total phospholipids so obtained under the conditions similar to EXAMPLE 1.

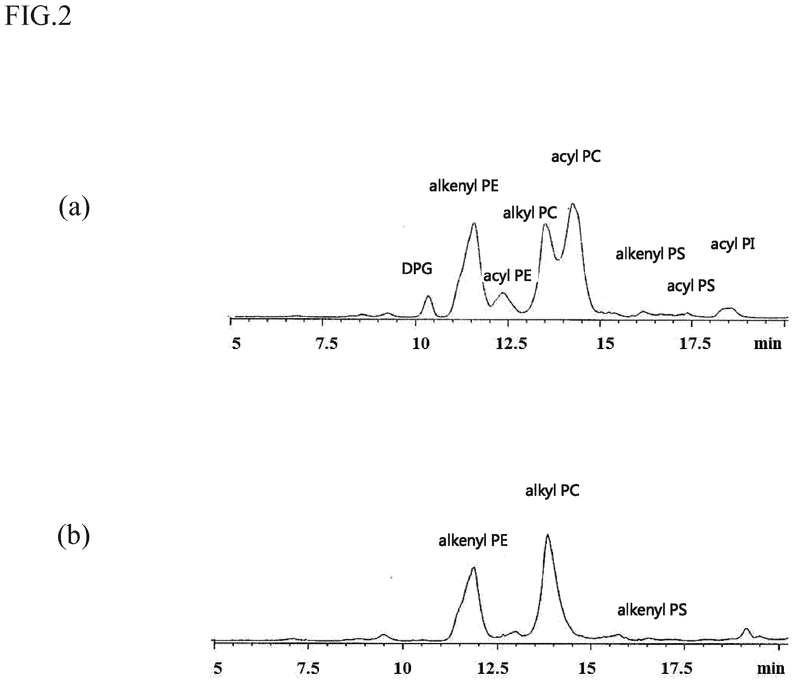

The result is shown in FIG. 2(a).

### (3) Purification of ether phospholipids by enzyme treatment and extraction processing of total phospholipids

By using the similar method of EXAMPLE 1, the ether phospholipids are purified by preparing corbicula-derived ether phospholipids from total lipids (purity: approximately 90%).

### (4) HPLC analysis of purified corbicula-derived ether phospholipids

HPLC analysis was provided on the corbicula-derived ether phospholipids so obtained under the conditions similar to EXAMPLE 1.

The result is shown in FIG. 2(b).

As a consequence, we could obtain highly-purified ether phospholipids derived from corbiculae, where the purity was approximately 90%.

Therefore, according to the present invention, it is clear that it enables to produce ether phospholipids contained in organisms of high purity by an easy process.

### EXAMPLE 3

### (Production of ether phospholipids derived from chicken breast)

### (1) Extraction of extraction of total chicken breast phospholipids

Except that chicken breast was used instead of clam, total phospholipids were obtained by the method similar to EXAMPLE 1.

### (2) HPLC analysis of chicken breast-derived total phospholipids

HPLC analysis was provided on the total phospholipids so obtained under the conditions similar to EXAMPLE 1.

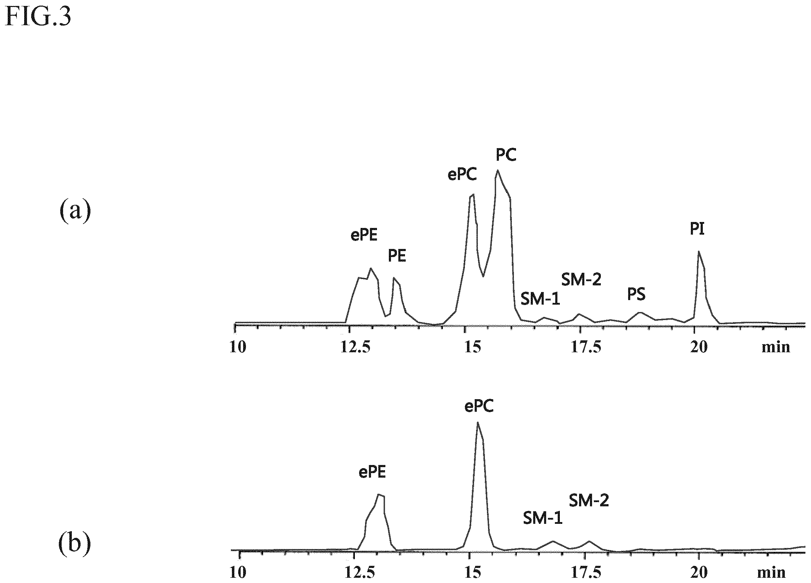

The result is shown in FIG. 3(a).

In this figure, references are as below:
ePE: Ethanolamine ether phospholipid
ePC: Choline ether phospholipid

### (3) Purification of ether phospholipids by enzyme treatment and extraction processing of total phospholipids

By using the similar method of EXAMPLE 1, the ether phospholipids are purified by preparing chicken breast-derived ether phospholipids from total lipids (purity: approximately 92%).

### (4) HPLC analysis of purified chicken breast-derived ether phospholipids

HPLC analysis was provided on the chicken breast-derived ether phospholipids so obtained under the conditions similar to EXAMPLE 1.

The result is shown in FIG. 3(b).

As a consequence, we could obtain highly-purified ether phospholipids derived from chicken breast, where the purity was approximately 92%.

Therefore, according to the present invention, it is clear that it enables to produce ether phospholipids contained in organisms of high purity by an easy process.

### EXAMPLE 4

### (Analysis of Polyunsaturated Fatty Acids within Total Fatty Acids of Ether Phospholipids)

An analysis using HPLC was provided with regards to the ratio of polyunsaturated fatty acids (EPA, DHA and ARA) within total fatty acids contained in bivalve (clam, corbicula)-derived ether phospholipids and chicken-derived ether phospholipids, according to the following method.

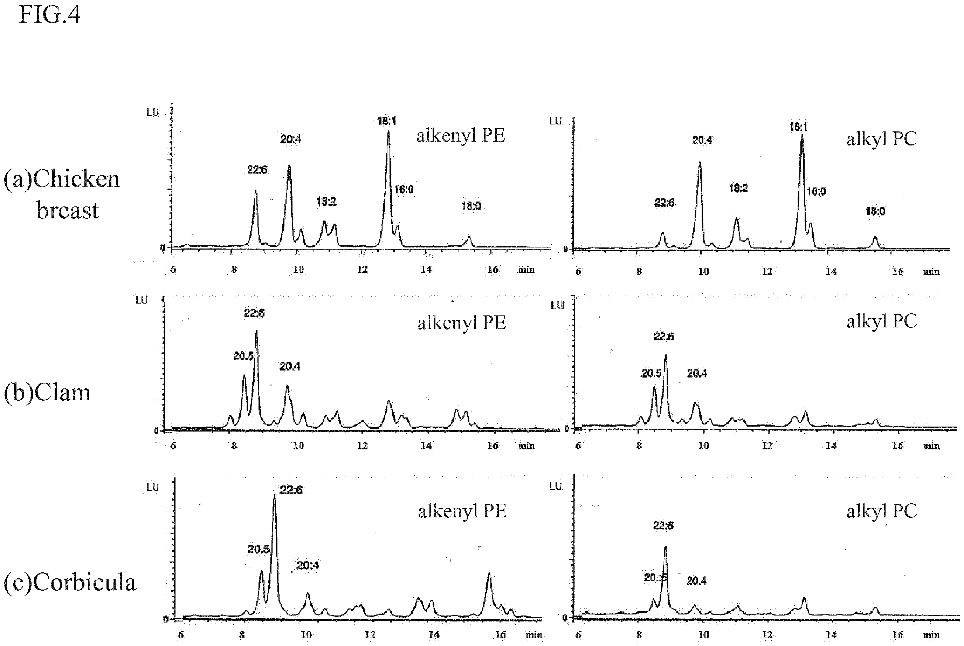

The results are shown in TABLES 3 and 4 and FIG. 4.

### (Hydrolysis of various ether phospholipids)

Various kinds of ether phospholipids were separately obtained and dried, which was then hydrolyzed with methanol solution of 0.5N potassium hydroxide. Accordingly, free fatty acids were obtained.

### (Label for free fatty acids from various bivalve-derived ether phospholipids)

Free fatty acids produced by hydrolysis from the ether phospholipids were then labeled by 0.05% 9-anthryldiazomethane (ADAM).

### (Analysis of fatty acids)

HPLC analysis was provided on the fatty acids so labelled under the following conditions.

### <Conditions of HPLC>:

| | |
|---|---|
| Device used: | Agilent HPLC 1100 Series (Agilent Technologies) |
| Column: | Ultrasphere 100 RP-18e (Merck); |
| Flow rate: | 0.8ml/min |
| Detection: | Fluorometric detector |

### Mobile phases:

(A) Acetonitrile
(B) Ethanol
(C) Hexane

TABLE 2 shows a liquid composition in terms of time zone and mobile phases (A) to (C).

**TABLE 2**

| Time (min.) | Mobile phase (A) (%) | Mobile phase (B) (%) | Mobile phase (C) (%) |
|---|---|---|---|
| 0 | 100 | 0 | 0 |
| 16 | 44 | 32 | 24 |
| 16.1 | 100 | 0 | 0 |
| 24 | 100 | 0 | 0 |

**TABLE 3: Polyunsaturated Fatty Acids within Total Fatty Acids of Various Ether Phospholipids**

| | Clam Alkenyl PE | Corbicula Alkenyl PE | Chicken Breast Alkenyl PE |
|---|---|---|---|
| EPA (20:5) | 11.1 | 12.1 | - |
| DHA (22:6) | 31.8 | 23.2 | 14.7 |
| ARA (20:4) | 8.5 | 15.2 | 23.6 |

| | | | |
|---|---|---|---|
| EPA: eicosapentaenoic acid DHA: docosahexaenoic acid ARA: arachidonic acid | | | |

**TABLE 4: Polyunsaturated Fatty Acids within Total Fatty Acids of Various Ether Phospholipids**

| | Clam Alkyl PC | Corbicula Alkyl PC | Chicken Breast Alkenyl PC |
|---|---|---|---|
| EPA (20:5) | 10.5 | 15.1 | - |
| DHA (22:6) | 37.4 | 25.5 | 5.6 |
| ARA (20:4) | 8.3 | 8.7 | 29.2 |

| | | | |
|---|---|---|---|
| EPA: eicosapentaenoic acid DHA: docosahexaenoic acid ARA: arachidonic acid | | | |

According to TABLES 3 and 4 as well as FIG. 4, it is found that bivalve (clam, corbicula)-derived ether phospholipids are bound, at the *sn-*2 position, to a large amount of eicosapentaenoic acid (EPA), docosahexaenoic acid (DHA) and arachidonic acid (ARA).

On the other hand, with regards to chicken breast-derived ether phospholipids that were analyzed simultaneously by the same method as the bivalve-derived ether phospholipids, both PE and PC are alkenyl phospholipids and no alkyl PC is present.

Therefore, by comparing the compositions of fatty acids, it was demonstrated that bivalve-derived ether phospholipids contain EPA and DHA in a larger amount.

EPA and DHA are decreased in brain diseases such as Alzheimer's disease, Parkinson disease, depression and schizophrenia, metabolic diseases such as diabetes, various infectious diseases, and immune disorders. Ether phospholipids derived from dietary bivalves function to supplement EPA and DHA.

While bivalve-derived ether phospholipids contain EPA and DHA in a large amount, chicken breast-derived ether phospholipids do not contain EPA at all and do contain DHA yet in a small amount.

Therefore, according to the present invention, since bivalve-derived ether phospholipids contain a larger amount of eicosapentaenoic acid (EPA), docosahexaenoic acid (DHA) and arachidonic acid (ARA), it is expected to achieve superior effects as compared to the conventional ether phospholipids, in particular ether phospholipids derived from chicken breast.

### INDUSTRIAL UTILITY

According to the present invention, it enables to produce ether phospholipids of high purity by an easy process, that are effective for treating and improving brain diseases such as Alzheimer's disease, Parkinson disease, depression and schizophrenia, metabolic diseases such as diabetes, various infectious diseases, and immune disorders. Therefore, the present invention may be applied widely in the medicinal industry.

## Claims

1. A method for producing ether phospholipids from organisms comprising:
(A) a step of extracting total lipids with a non-polar solvent and branched alcohol mixture; and
(B) a step of reacting the total lipids obtained by the step (A) with phospholipase A1 to decompose mixed diacyl-glycerophospholipids.

2. The method for producing ether phospholipids according to Claim 1, wherein the non-polar organic solvent and branched alcohol mixture is hexane/isopropanol mixture.

3. The method for producing ether phospholipids according to Claim 1 or 2 including a step of purifying the resulting mixture after the step (B) is provided.

4. The method for producing ether phospholipids according to Claim 3, wherein the purification step is made by solution partitioning.

5. The method for producing ether phospholipids according to Claims 4, wherein the solution partitioning is made by extracting lipids with hexane followed by separating with acetone or water.
